# EUROPEAN PATENT APPLICATION

(11) **EP 3 858 215 A1**
(43) Date of publication of application: **04.08.2021**
(21) Application number: 19866114.2
(22) Date of filing: 11.09.2019
(51) Int. Cl.: A61B 1/00, A61B 1/06, G01N 21/64

(54) **FLUORESCENT OBSERVATION CAMERA SYSTEM**

(30) Priority: 25.09.2018 JP 2018179045
(71) Applicant: Mizuho Corporation, Bunkyo-ku Tokyo 113-0033 (JP)
(72) Inventor: FUJITA, Junichi, Gosen-shi Niigata 959-1821 (JP); NAKASHIMA, Shinichi, Gosen-shi Niigata 959-1821 (JP); KITANI, Ichiro, Tokyo 113-0033 (JP)
(74) Representative: Hafner & Kohl PartmbB
(86) International application number: PCT/JP2019/035666
(87) International publication number: WO 2020/066610

(57) **Abstract**

Provided is a novel fluorescent observation camera system capable of managing a variety of fluorescent reagents with a single device and of detecting different types of tissues at the same time. The system comprises a camera unit 1 for imaging a subject, wherein the camera unit 1 further comprises: a lens 2 to collect infrared light and visible light from the subject; an illumination means 3 that is circumferentially arranged around the lens 2 and comprises a white light source and at least two types of excitation light sources, said illumination means 3 being configured to simultaneously emit two types of excitation lights; a filter 4 that removes the excitation light that has been reflected from the subject and passed through the lens 2; a beam splitter 5 to separate the infrared light and the visible light that have passed through the filter 4, from each other; an infrared light imaging component 6 to image the infrared light separated by the beam splitter; and a visible-light imaging component 7 to image the visible light separated by the beam splitter.

## Description

### [Technical field]

The present invention relates to a fluorescent light observation camera system for observing a tissue in a living body in real time.

### [Background art]

As an example of such device for observing a tissue in a living body in real time, an imaging device as described in patent document 1 is known. This imaging device photographs a composite image of a visible-light image and an infrared-light image using a single imaging component from a subject such as human body to which indocyanine green (ICG hereafter), emitting infrared-light, is vascularly administered as a contrast medium. In addition, patent document 2 discloses an optical living body imaging system in which the system is configured to simultaneously observe fluorescence and also observation light whose spectra in the excitation-light band are eliminated. Patent document 3 discloses an infrared fluorescence observation device that attenuates excitation-light spectrum in the light composed of an excitation light reflected from a subject and a fluorescence emitted from ICG excited by the excitation light. This device performs photographing with the light in which the excitation light component is attenuated, and also performs photographing with the light from which the fluorescence is eliminated. The device then takes a difference between the respective images generated on the basis of the pixel signals obtained in each photographing to thereby obtain an image of the subject taken only with fluorescence. Also, patent document 4 discloses an imaging system that pictures a reflected light image and a fluorescent image from a surgical field.

These prior art references are all configured to irradiate a visible light and an excitation light to image a fluorescent image from the subject. However, these devices provide only one type of excitation light source, and are compatible only with a respective excitation wavelength for a specific one type of fluorescence reagent.

Meanwhile, as fluorescence reagents for observing a tissue in a living organism, 5-aminolevulinic acid (5-ALA hereafter) and fluoresceine are known on top of the above-mentioned ICG. Here, ICG utilizes an excitation light of 760 nm to emit fluorescence at 830 nm, which is used for determining hepatic function, ophthalmic disorder, or breast cancer. 5-ALA utilizes an excitation light of 405 nm to emit fluorescence at 635 nm, which is used for detecting a cancer cell or a brain tumor. Fluoresceine utilizes an excitation light of 494 nm to emit fluorescence at 521 nm, which is used for determining ophthalmic disorder or corneal abnormality.

Unfortunately, as described above, these prior arts provide light sources that are compatible only with a respective excitation wavelength for a specific one type of fluorescence reagent. For this reason, single-purpose devices are individually required for managing a variety of fluorescence reagents. Further, as the target tissue within a living body, which is subjected to the detection, varies depending on the type of fluorescence reagent, it has never been possible in the prior arts to simultaneously detect different types of tissues with a single device. A significant contribution to the efficiency in surgery or diagnosis would therefore be expected if there is provided a device that can manage a variety of fluorescence reagents with a single device and detect different types of tissues at the same time.

### [Prior art documents]

### [Patent documents]

Patent document 1: JP4971816(B2)
Patent document 2: JP-A-2016-87062
Patent document 3: WO2016/194101(A1)
Patent document 4: US8229548(B2)

### [Summary of the invention]

### [Problems to be solved by the invention]

The object of the present invention is therefore to provide a novel fluorescent observation camera system that can manage a variety of fluorescent reagents with a single device and detect different types of tissues at the same time.

### [Means to solve the problems]

The inventive fluorescent observation camera system may comprise: a camera unit for imaging a subject; and a controller unit for controlling the camera unit, wherein the camera unit comprises: a lens to collect infrared light and visible light from the subject; an illumination means that is circumferentially arranged around the lens and comprises a white light source and at least two types of excitation light sources, said illumination means being configured to simultaneously emit two types of excitation lights; a filter that removes the excitation light that has been reflected from the subject and passed through the lens; a beam splitter to separate the infrared light and the visible light that have passed through the lens, from each other; an infrared light imaging component to image the infrared light separated by the beam splitter; and a visible-light imaging component to image the visible light separated by the beam splitter.

The illumination means may comprise at least three types of excitation light sources and be configured to simultaneously emit any two types of excitation lights.

The controller unit may comprise: a controlling means for controlling turning on or off the white light source, the excitation light sources and the filter; and an image processing means that processes signals from the infrared light imaging component and the visible-light imaging component to produce an infrared-light image, a visible-light image and/or a composite image of the infrared and visible light images.

The illumination means may comprise three types of excitation light sources respectively compatible with the excitation wavelengths of indocyanine green, 5-aminolevulinic acid and fluoresceine.

### [Effects of the invention]

According to the inventive fluorescent observation camera system, a variety of fluorescent reagents can be managed with a single device, and different types of tissues can be simultaneously detected at the same time.

### [Brief description of the drawings]

[FIG. 1] is a schematic view showing the appearance of the fluorescent observation camera system according to an exemplary embodiment of the present invention.
[FIG. 2] is a front view of the fluorescent observation camera system according to an exemplary embodiment of the present invention.
[FIG. 3] is a block diagram illustrating optical and electrical configurations of the fluorescent observation camera system according to an exemplary embodiment of the present invention.
[FIG. 4A] is an explanatory drawing illustrating a lighting pattern of the light sources of the illumination means according to an exemplary embodiment of the inventive fluorescent observation camera system.
[FIG. 4B] is an explanatory drawing illustrating a lighting pattern of the light sources of the illumination means according to an exemplary embodiment of the inventive fluorescent observation camera system.
[FIG. 4C] is an explanatory drawing illustrating a lighting pattern of the light sources of the illumination means according to an exemplary embodiment of the inventive fluorescent observation camera system.
[FIG. 4D] is an explanatory drawing illustrating a lighting pattern of the light sources of the illumination means according to an exemplary embodiment of the inventive fluorescent observation camera system.
[FIG. 4E] is an explanatory drawing illustrating a lighting pattern of the light sources of the illumination means according to an exemplary embodiment of the inventive fluorescent observation camera system.
[FIG. 4F] is an explanatory drawing illustrating a lighting pattern of the light sources of the illumination means according to an exemplary embodiment of the inventive fluorescent observation camera system.
[FIG. 4G] is an explanatory drawing illustrating a lighting pattern of the light sources of the illumination means according to an exemplary embodiment of the inventive fluorescent observation camera system.

### [Mode for carrying out the invention]

Exemplary embodiments of the inventive fluorescent observation camera system will be described hereunder with reference to the appended drawings.

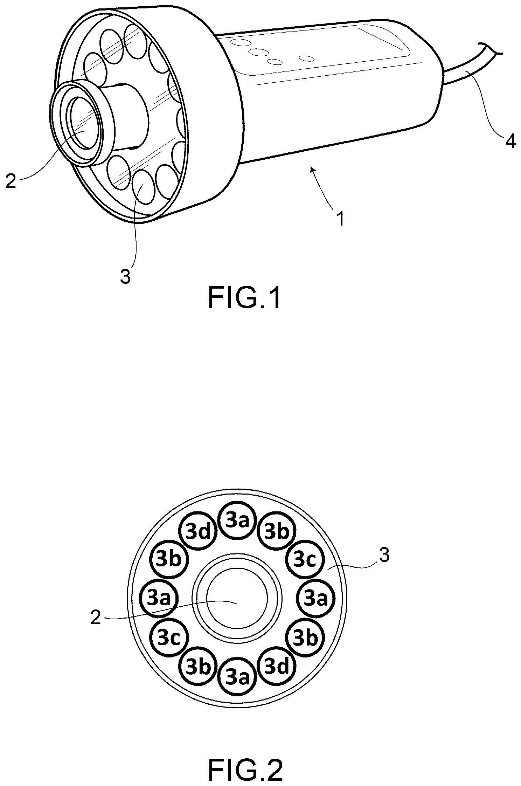

In FIG. 1 illustrating a schematic view of the fluorescent observation camera system according to an exemplary embodiment of the present invention, numeral 1 refers to a camera unit for imaging a subject. At an anterior surface of the camera unit 1, there is provided a lens 2 to collect infrared light and visible light from the subject. An illumination means 3 is circumferentially arranged around the lens 2 such that the illumination means 3 is configured to irradiate light toward the anterior direction in which the lens 2 is directed. The posterior of the illumination means 3 is formed to have a substantially rectangular cross section for allowing it to be easily gripped by a user. Further, a wire 4 is provided at the posterior portion of the camera unit 1 for electrically connecting it to a controller unit 21 (which will be described later).

In FIG. 2 illustrating the front view of the camera unit 1, the illumination means 3 has 12 light-emitting diodes (LEDs) that are circumferentially arranged at regular intervals. Among the 12 LEDs, four of them are near-infrared LEDs 3a provided as an excitation light source to emit near-infrared light for exciting indocyanine green (ICG), another four of them are first visible-light LEDs 3b provided as a first excitation light source to emit 405nm visible light for exciting 5-aminolevulinic acid (5-ALA), two of them are second visible-light LEDs 3c provided as an excitation light source to emit 470nm visible light for exciting fluoresceine; and the remaining two of them are white LEDs 3d provided as a white light source to emit white light for auxiliary illumination. These four types of LEDs are configured in a manner such that one can selectively perform single mono-light emission per each type of LED; or simultaneous light emission combining any multiple types of LED. That is, any two or more types of excitation lights can be emitted at the same time. In any of these light emission modes, the light sources of the same type are arranged at positions furthest away from each other for allowing the subject to be evenly irradiated.

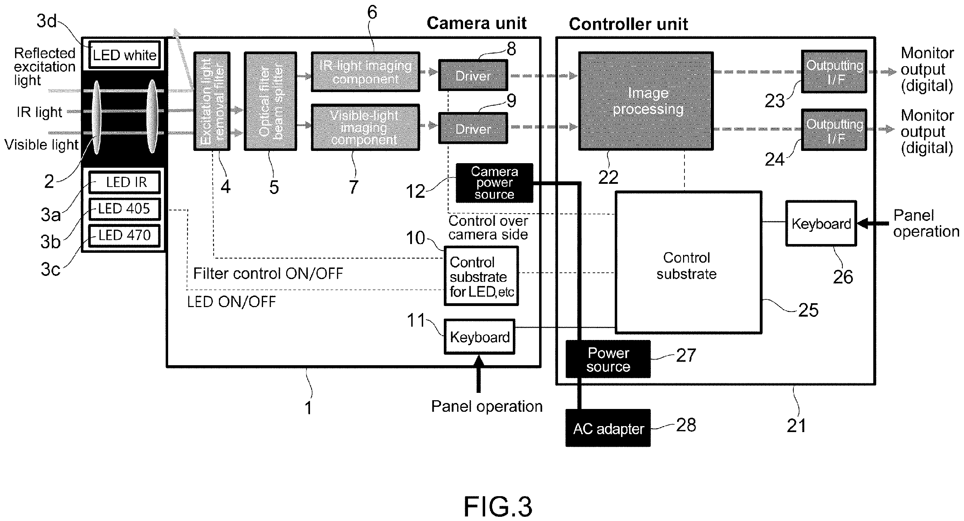

In FIG. 3 which is a block diagram illustrating optical and electrical configurations, the light from the near-infrared LEDs 3a, the first visible-light LEDs 3b, the second visible-light LEDs 3c and the white LEDs which are respectively provided in the camera unit 1 is cast/turned back from the subject, and then collected by the lens 2 as an infrared light and a visible light. The filter 4 removes the spectra of the excitation lights from the infrared and visible lights collected by the lens 2, and the beam splitter 5 then separates the transmitted infrared and visible lights that have passed through the filter 4 from each other. The infrared light separated by the beam splitter 5 is imaged by an infrared-light imaging component 8. The visible light separated by the beam splitter 5 is imaged by a visible-light imaging component 9. These are respectively converted into corresponding electric signals. These electric signals are then output by the respective drivers 8 and 9 to the external.

Numeral 21 refers to a controller unit for controlling the camera unit 1 in which the signals that have been output from the camera unit 1 are input into an image processing means 22. The image processing means 22 processes the input signals to produce an infrared light image, a visible-light image or a composite image of the infrared and visible-light images. The corresponding signals of these images are output through outputting interfaces 23 and 24 to a monitor to display the images on the monitor.

The controller unit 21 includes the image processing means 22 and a control substrate 25 provided as a controlling means for controlling the driving units 8, 9 and a control substrate 10 in the camera unit 1. Operation of the control substrate 25 may be switched by panel operations into a keyboard 11 provided in the camera unit 1 and into a keyboard 26 provided in the controller unit 21. Further, the control substrate 10 of the camera unit 1 is configured to control turning on or off the near-infrared LEDs 3a, the first visible-light LEDs 3b, the second visible-light LEDs 3c and the white LEDs 3d based on the instructions from the control substrate 25 in the controller unit 21. In the meantime, the control substrate 10 is also configured to control turning on or off the filter 4 in association with the switching of these LEDs.

In addition, a power source 12 of the camera unit 1 is supplied from a power source 27 of the controller unit 21, which is originally supplied from an AC adapter 28.

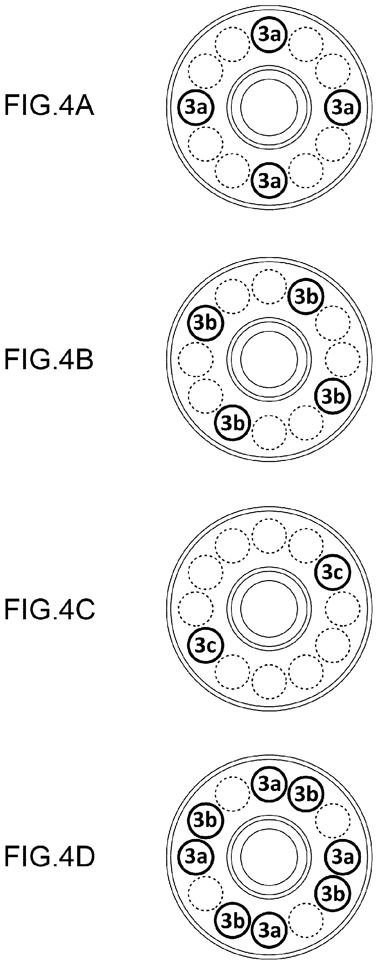

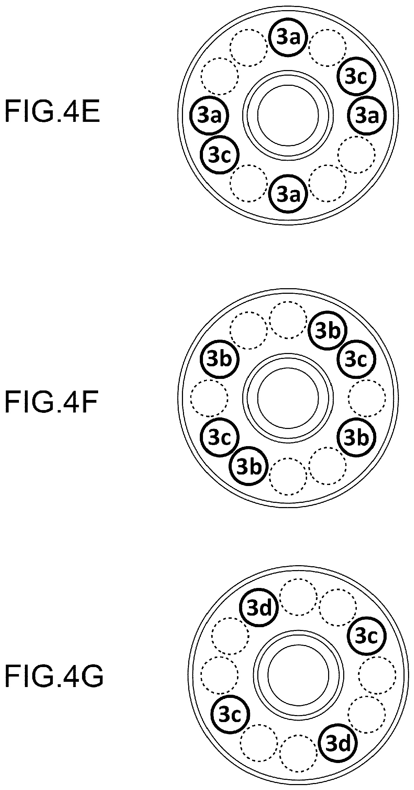

Next, with reference to FIG. 4, there will be described lighting patterns of the near-infrared LED 3a, the first visible-light LEDs 3b, the second visible-light LEDs 3c and the white LEDs 3d which are light sources of the illumination means 3 of the camera unit 1.

FIG. 4A is an explanatory drawing illustrating a lighting pattern in which only the near-infrared LEDs 3a configured as an excitation light source of ICG are turned on, where the solid-line circles represent LEDs that are turned on, while the broken-line circles represent LEDs that are turned off. In this lighting pattern, a near-infrared fluorescence of ICG with a peak at the wavelength of 830 nm is captured by the infrared-light imaging component 6, which is then displayed on the monitor.

FIG. 4B is an explanatory drawing illustrating a lighting pattern in which only the first visible-light LEDs 3b configured as an excitation light source of 5-ALA are turned on. In this lighting pattern, a red fluorescence of 5-ALA with a peak at the wavelength of 635 nm is captured by the visible-light imaging component 9, which is then displayed on the monitor.

FIG. 4C is an explanatory drawing illustrating a lighting pattern in which only the second visible-light LEDs 3c configured as an excitation light source of fluoresceine are turned on. In this lighting pattern, a green fluorescence of fluoresceine with a peak at the wavelength of 521 nm is captured by the visible-light imaging component 9, which is then displayed on the monitor.

FIG. 4D is an explanatory drawing illustrating a lighting pattern in which both the near-infrared LEDs 3a configured as an excitation light source of ICG and the first visible-light LEDs 3b configured as an excitation light source of 5-ALA are simultaneously turned on at the same time. In this lighting pattern, a near-infrared fluorescence of ICG with a peak at the wavelength of 830 nm is captured by the infrared light imaging component 6 and then displayed on the monitor as a black- and-white image, while a red fluorescence of 5-ALA with a peak at the wavelength of 635 nm is captured by the visible-light imaging component 9 and then displayed on the monitor.

FIG. 4E is an explanatory drawing illustrating a lighting pattern in which both the near-infrared LEDs 3a configured as an excitation light source of ICG and the second visible-light LEDs 3c configured as an excitation light source of fluoresceine are simultaneously turned on at the same time. In this lighting pattern, a near-infrared fluorescence of ICG with a peak at the wavelength of 830 nm is captured by the infrared light imaging component 6 and then displayed on the monitor, while a green fluorescence of fluoresceine with a peak at the wavelength of 521 nm is captured by the visible-light imaging component 9 and then displayed on the monitor.

FIG. 4F is an explanatory drawing illustrating a lighting pattern in which both the first visible-light LEDs 3b configured as an excitation light source of 5-ALA and the second visible-light LEDs 3c configured as an excitation light source of fluoresceine are simultaneously turned on. In this lighting pattern, the visible-light imaging component 9 captures a red fluorescence of 5-ALA with a peak at the wavelength of 635 nm and a green fluorescence of fluoresceine with a peak at the wavelength of 521 nm, which are then displayed on the monitor.

FIG. 4G is an explanatory drawing illustrating a lighting pattern in which both the second visible-light LEDs 3c and the white LEDs 3d configured as a white light source that emits white light for supplementary lighting are simultaneously turned on at the same time. In this lighting pattern, a light emission at a region close to 470 nm having a weak sensitivity in the emission spectra of white LEDs 3d will be supplemented with the light emission by the second visible-light LEDs 3c that emit visible light at the wavelength of 470 nm to achieve an auxiliary illumination which is natural in hue.

As shown, the lightings of near-infrared LEDs 3a, the first visible-light LEDs 3b, and the second visible-light LEDs 3c may be combined, and the wavelength regions to be removed by the filter 4 may be suitably selected to remove excitation lights that have been reflected from a subject in order to, for example, observe the fluorescence of ICG and that of 5-ALA at the same time, observe the fluorescence of ICG and that of fluoresceine at the same time, or observe the fluorescence of 5-ALA and that of fluoresceine at the same time. Alternatively, three types of fluorescence may be simultaneously observed at the same time. For example, in this case, all of the LEDs in the illumination means 3 may be turned on, and then the three types of excitation lights reflected from the subject may be removed by the filter 4 to observe the three types of fluorescence at the same time.

Here, the filter 4 is configured such that multiple filters are combined to be individually turned on or off to remove excitation lights at multiple wavelength regions which have been reflected from a subject. Also, the lighting patterns of the illumination means 3 and the combination of wavelength regions to be removed by the filter 4 are preprogrammed into the control substrate 25 in the controller unit 21 such that these are easily controlled by simply selecting a desired lighting pattern with panel operations of the keyboards 11 and 26.

As shown in the above, the fluorescent observation camera system of the present invention may comprise: a camera unit 1 for imaging a subject; and a controller unit 21 for controlling the camera unit 1, wherein the camera unit 1 comprises: a lens 2 to collect infrared light and visible light from the subject; an illumination means 3 that is circumferentially arranged around the lens 2 and comprises white LEDs3d, configured as a white light source, and near-infrared LEDs 3a, first visible-light LEDs 3b and second visible-light LEDs 3c which are respectively configured as at least two types of excitation light sources, said illumination means 3 being configured to simultaneously emit two types of excitation lights at the same time; a filter 4 that removes the excitation light that has been reflected from the subject and passed through the lens 2; a beam splitter 5 to separate the infrared light and the visible light that have passed through the filter 4 from each other; an infrared-light imaging component 6 to image the infrared light separated by the beam splitter; and a visible-light imaging component 7 to image the visible light separated by the beam splitter 5.

The illumination means 3 may comprise the near-infrared LEDs3a, the first visible-light LEDs 3b and the second visible-light LEDs 3c which are configured as at least three types of excitation light sources and be configured to simultaneously emit any two types of excitation lights.

The controller unit 21 may comprise: a control substrate 25 configured as a controlling means for controlling turning on or off the white LEDs 3 d, the near-infrared LEDs3a, the first visible-light LEDs 3b, the second visible-light LEDs 3c and the filter 4; and an image processing means 22 that processes signals from the infrared light imaging component 6 and the visible-light imaging component 7 to produce an infrared light image, a visible-light image and/or a composite image of the infrared and visible-light images.

Further, the illumination means 3 may comprise three types of excitation light sources respectively compatible with the excitation wavelengths of indocyanine green, 5-aminolevulinic acid and fluoresceine.

The fluorescent observation camera system of the present invention can manage a variety of fluorescent reagents with a single device and detect different types of tissues at the same time.

### [List of reference numerals]

- 1: camera unit
- 2: lens
- 3: illumination means
- 3d: white LED (white light source)

- 3a: near-infrared LED (excitation light source)
- 3b: first visible-light LED (excitation light source)
- 3c: second visible-light LED (excitation light source)
- 4: filter
- 5: beam splitter
- 6: infrared light imaging component
- 7: visible-light imaging component
- 21: controller unit
- 25: control substrate (controlling means)
- 22: image processing means

## Claims

1. A fluorescent observation camera system comprising:
a camera unit for imaging a subject; and
a controller unit for controlling the camera unit,
wherein the camera unit comprises:
a lens to collect infrared light and visible light from the subject;
an illumination means that is circumferentially arranged around the lens and comprises a white light source and at least two types of excitation light sources, said illumination means being configured to simultaneously emit two types of excitation lights;
a filter that removes the excitation light that has been reflected from the subject and passed through the lens;
a beam splitter to separate the infrared light and the visible light that have passed through the filter, from each other;
an infrared light imaging component to image the infrared light separated by the beam splitter; and
a visible-light imaging component to image the visible light separated by the beam splitter.

2. The fluorescent observation camera system according to claim 1, wherein the illumination means comprises at least three types of excitation light sources and is configured to simultaneously emit any two types of excitation lights.

3. The fluorescent observation camera system according to claim 1 or 2, wherein the controller unit comprises:
a controlling means for controlling turning on or off the white light source, the excitation light sources, and the filter; and
an image processing means that processes signals from the infrared light imaging component and the visible-light imaging component to produce an infrared light image, a visible-light image and/or a composite image of the infrared and visible-light images.

4. The fluorescent observation camera system according to claim 2 or 3, wherein the illumination means comprises three types of excitation light sources respectively compatible with the excitation wavelengths of indocyanine green, 5-aminolevulinic acid and fluoresceine.
